# EUROPEAN PATENT SPECIFICATION

(11) **EP 2 442 734 B1**
(45) Date of publication and mention of the grant of the patent: **05.08.2020**
(21) Application number: 10742244.6
(22) Date of filing: 14.06.2010
(51) Int. Cl.: A61B 17/32

(54) **DEVICE FOR ENDOSCOPIC RESECTION OR REMOVAL OF TISSUE**
VORRICHTUNG FÜR ENDOSKOPISCHE RESEKTION ODER ENTFERNUNG VON GEWEBE
DISPOSITIF POUR RÉSECTION OU EXTRACTION ENDOSCOPIQUE DE TISSUS

(30) Priority: 16.06.2009 CH 941092009
(43) Date of publication of application: 25.04.2012
(73) Proprietor: FRII S.A., 1260 Luxembourg (LU)
(72) Inventor: RIVA, Raffaele, 6901 Lugano (CH)
(74) Representative: Fabiano, Piero
(86) International application number: PCT/IB2010/001414
(87) International publication number: WO 2010/146432

(56) References cited:
- WO-A1-96/29014
- US-A- 4 050 528
- US-A- 5 505 210
- US-A- 5 796 188
- US-A- 5 849 023
- US-A1- 2004 092 992
- US-A1- 2008 208 233
- US-A1- 2008 234 715

## Description

The present invention relates to a device for treatments of endoscopic resection/removal of tissues. In particular, the invention relates to an endoscopic device for treatments of resection of soft tissue or osseus tissue (with relative removal of the fragments produced by such an action).

As an alternative to the traditional surgery, which requires a relatively wide incision to access to the surgical site inside the human body, the endoscopic procedures utilize natural accesses or as an alternative the creation of small portals (minimal tissular incisions); therefore often reference is made to the endoscopic surgery with the term of mini-invasive surgery. The two main advantages of the endoscopic surgery are the more rapid healing of the tissues after the surgical operation and the lesser exposition of internal tissues to the risk of infection. The technological developments in this surgical field, also defined "closed", have led to the realization of many minimally invasive instruments, as the access to the surgical site is made through one or more portals. Such instruments must be sufficiently elongated and smooth to permit the entrance and the use with a minimum trauma for the surrounding tissues.

A portion of the instrument, usually indicated as "distal portion", is so conceived in order to have access to the surgical site; the opposite portion, usually indicated as "proximal portion", remains at the outside of the body of the patient. The distal portion of the instrument is typically provided for treating the tissue with which it comes in contact, its shape and its dimensions being therefore properly studied in function of the particular surgical operation to which it is destined.

The proximal portion is instead provided with a mechanism to control from the outside of the body of the patient the above function. The motorized endoscopic surgical instruments, used in the "closed" surgery, often identified as endoscopic "shavers", are typically made by a pair of coaxial tubular concentrically disposed elements: an external element ending distally with an aperture or "cutting window" and a rotary internal element having a sharp surface at the cutting window. The rotary action of the internal tubular element produces by abrasion the removal or the finishing of the tissue, this process being defined as "resection".

As in each surgical action, also in the endoscopic surgery the presence of two well distinct fields is provided: the sterile field, the one in close contact with the patient, whereby the surgeon will perform his operation, and the one definitely separated from the patient and from any object coming in contact with it. Only suitably treated personnel and instruments can access to the sterile field (sterilization processes for the instruments, washing pre-operatory processes and adoption of protective aids for the personnel, as gloves and coats); all that can not enter in contact with the sterile field must rigorously remain outside of it.

US2004/092992 discloses a disposable battery powered rotary tissue cutting instrument for soft tissue removal

US2007/0010823 describes a "shaver" for arthroscopic operations and a system for providing the suction and the irrigation during a medical procedure with the above "shaver".

US 5,669,921 describes a cutting device comprising:
- an elongated external tube having a proximal end, a distal end and at said proximal end a bushing to permit the attachment of the external tube to an electrically fed sleeve; and
- an elongated internal tube apt to be received in said external tube, having a proximal end, a distal end, an internal aperture at said distal end, a cutting tip
   and a bushing disposed at the proximal end, the bushing permitting the connection of the internal tube to guide means for the cutting device.

The Applicant has noted that in the endoscopic "shavers" actually existing and/or in those described above the internal tubular element is brought in rotation and controlled by a handpiece having internally a small electric motor: the actuation and control are made either by pushbuttons positioned on the handpiece itself or by pushbuttons positioned on a pedal board. In both cases the power and the control signals arrive at the handpiece through a wire connected with an external bracket. This "bracket" is usually disposed on a trolley sufficiently distant from the operation field in order not to contaminate the sterile field. The handpiece (which comes in contact with the sterile field) undergoes a sterilization treatment before each surgical operation; the bracket having to remain out of each contact with the sterile zone, is housed out of the aforesaid field; in the actually existing systems, a connection wire is provided between handpiece and "bracket". Such connection wire before each use is treated in order to render it completely sterile and at the preparation of the surgical operation it is assembled from one side with the (sterile) handpiece and from the other side with the (non sterile) bracket. In the actually existing "shavers" the handpiece is made of a metallic material, so it has a non negligeable weight, and the connection wire has a weight and encumbrance such to limit the handling of the operator.

The personnel of the operation room which is responsible for the treatment and the management of the instrument at the end of each operation has to perform the washing (with suitable disinfectants and detergents) and then the sterilizing of the resterilizable parts (handpiece and wire); the cleaning and the sterilization negatively affect the useful life of the sterilizable components.

The personnel of the operation room must further perform the storing in suitable containers which guarantee the sterility, with a consequent waste of time and space consumption.

Nevertheless the personnel of the operation room must perform the maintenance of the non sterilizable components, i.e. the bracket and the pedal board if present, by making periodical inspections which can require more complex technical interventions by qualified personnel.

The Applicant has found that with a device for treatments of endoscopic resection/removal of the tissues providing at least an essential portion of the disposable device, it can be avoided that the operation personnel of the treatment and the management of the device perform at the end of each operation the washing (with suitable disinfectants and detergents) and then the sterilizing of the handpiece and the wire.

In one of its first aspects, the invention concerns a device for treatments of endoscopic resection/removal of tissues comprising:
- a handpiece apt to be held by an user;
- an external tubular element comprising a proximal end, a distal end and a cutting aperture disposed at said distal end;
- an internal tubular element apt to be pivotally received in said external tubular element and comprising a proximal end, a distal end and a cutting tip at its distal end; wherein the proximal end of each of the external tubular element and internal tubular element are supported by the handpiece;
- guide means for rotating and/or oscillating said internal tubular element with respect to said external tubular element;
   wherein said guide means comprise an electric motor, a control unit and electric feeding means for said electric motor, said guide means being contained inside the handpiece; wherein said control unit comprises at least a main electronic circuit, to regulate the functions and the speed of the electric motor and of a plurality of pushbutton controls placed on the external surface of said guide means, in a position corresponding to pushbuttons provided on the external surface of the handpiece and characterized in that said guide means are contained inside a body insertable in a removable way inside said handpiece, said body being tight; the body axially extends inside the handpiece and only said handpiece is disposable.

With the term "disposable" in the present description and in the following claims it is obvious that the portion of the shaver so indicated is used for an only endoscopic operation or for part of an endoscopic operation at the end of which it is removed and no more utilized.

The personnel of the operation room making the treatment and management of the instrument has not to perform at the end of each operation the washing (with suitable disinfectants and detergents) and then the sterilizing of the resterilizable parts

The cleaning and sterilization of at least some parts of the shaver are avoided, with a relative lesser use of operation personnel, time and space.

The present invention, in the aforesaid aspect, can have at least one of the preferred features which are described in the following.

The guide means comprise electric feeding means for the electric motor.

According to a preferred aspect, the feeding means are contained inside the handpiece.

According to an alternative aspect, the feeding means are outside of the handpiece.

Only the handpiece is disposable. In this way, the more expensive portion of the device is recovered .

In an example not part of the present invention the guide means are contained inside a non mobile body, insertable in the handpiece.

The body is tight.

In order to permit a more rapid and easy extraction of the guide means with respect to the handpiece, the handpiece can comprise a distal portion supporting the external and the internal tubular element and a proximal portion engageable in a non mobile way with the distal portion.

The guide means also comprise a control unit contained inside the body. By providing the control unit inside the body, the manoevrability and the precision of the device are further improved.

The control unit comprises at least one electronic circuit to regulate the functions and the speed of the electric motor and of a plurality of pushbutton controls placed on the external surface of said guide means, in a position corresponding to pushbuttons of a flexible material provided on the external surface of the handpiece.

Preferably, the device can comprise a transmission group of the motion actuated by the electric motor for rotating the internal tubular element with respect to the external tubular element.

Advantageously, the transmission group of the motion can comprise at least a shaft pivotally supporting the internal tubular element and at least a control pinion which rotates the shaft, actuated by the electric motor.

Preferably, the device can comprise a suction and cooling circuit having a connection for a suction apparatus, and at least a duct guiding a cooling fluid inside the internal tubular element and a suction regulating device.

Advantageously the suction and cooling circuit has a heat exchange portion with said electric motor for limiting the heating of the electric motor. Preferably the suction regulating device comprises a tap and a lever for controlling the tap from outside.

Advantageously the electric motor is a brushless motor.

Further features and advantages of the invention will be more evident from the detailed description of some preferred but non exclusive embodiments, of a device for treatments of endoscopic resection/removal of tissues, according to the present invention.

Such description will be exposed here in the following with reference to the annexed drawings, given only for an indicating and therefore not limiting aim, in which:
- figure 1 is a schematic exploded view of a preferred embodiment of the device for treatments of endoscopic resection/removal of tissues, according to the present invention;
- figure 2 is a sectional side schematic view of the handpiece of the device for treatments of endoscopic resection/removal of tissues shown in figure 1;
- figure 3 is a sectional schematic view of an internal portion of the device in figure 1.

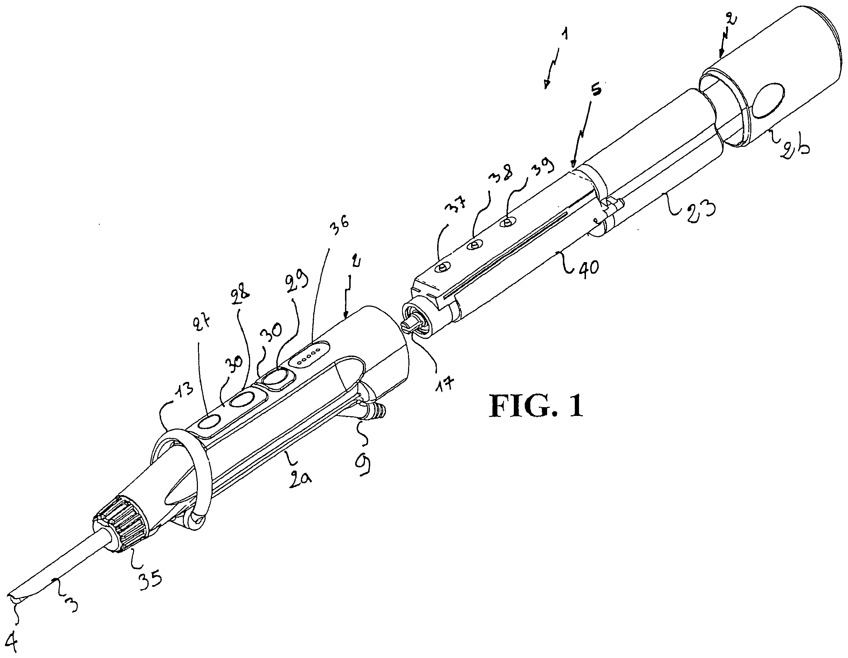

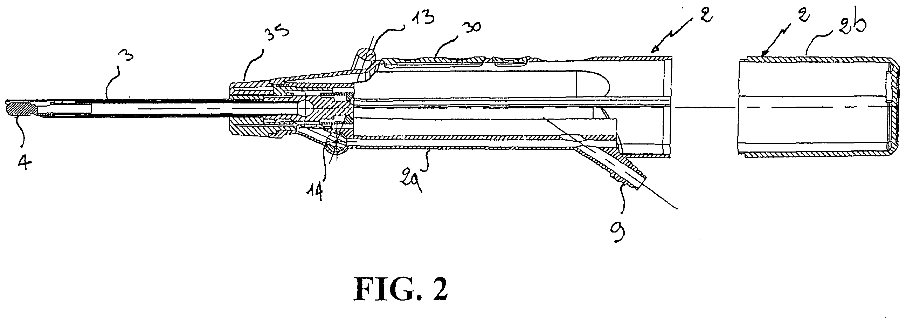

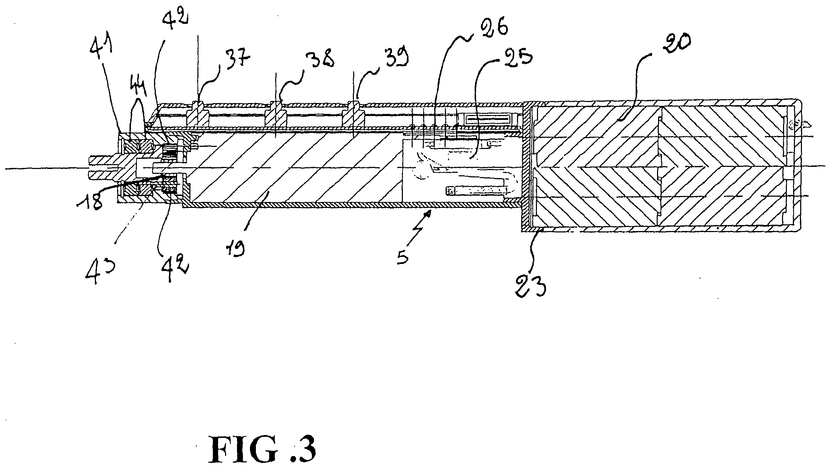

With reference to figures 1-3, a device for treatments of endoscopic resection/removal of tissues is indicated with the reference character 1.

The device for treatments of endoscopic resection/removal of tissues 1 comprises a handpiece 2 apt to be held by an user, an external tubular element 3, an internal tubular element 4 and guide means 5 for rotating and/or oscillating the internal tubular element 4 with respect to the external tubular element 3.

The external tubular element 3 comprises a proximal end, a distal end and an aperture and/or cutting window disposed at the distal end.

The internal tubular element is shaped and dimensioned in order to be pivotally housed in the external tubular element 3 and it comprises a proximal end, a distal end and a cutting tip at its distal end, facing the cutting window. The pivotal action of the internal tubular element 4 produces by abrasion the removal or the finishing of the tissue, and this process is defined as "resection".

The guide means 5 comprise an electric motor 19 and electric feeding means 20 for the electric motor 19. The guide means according to an important aspect of the invention are reusable, whereas the handpiece is disposable and single-used. To this aim the guide means 19 are contained inside a suitable body 40 which can be completely housed inside the handpiece 2. In this way, the more expensive portion of the device can be recovered.

In order to permit an easy and rapid extraction of the guide means 5 with respect to the handpiece 2, the handpiece 2 can comprise a distal portion 2a, supporting the external tubular elements 3 and the internal tubular elements 4 and a proximal portion 2b engageable in a non mobile way with the distal portion 2a.

The ability to realize some parts, such as the handpiece 2, the external tubular elements 3 and the internal tubular elements 4 which are disposable, i.e. single-used, reduces remarkably the problems related to the storing and sterilization of such parts by the personnel of the operation room.

Furthermore, the ability to insert inside body 40 some functional parts of the device, in particular the electric motor 19 and the electric feeding means 20, permits to increase remarkably the manoevrability and the precision of motion of the device according to the present invention, with respect to the cutting devices for arthroscopy present in the market.

The electric motor 19 is preferably a brushless type motor, but another type of electric motor with suitable dimensions and similar power could be apt to this aim. The motor 19 is able to rotate at a speed comprised between 400 and 4000 revolutions per minute.

The electric motor 19 is controlled by a unit that controls each function of the device 1, i.e. the starting, the rotation or the simple oscillation of the internal tubular element 4 with respect to the external tubular element 3 and the pivotal speed of the internal tubular element 4.

Also the control unit is provided inside said body.

The control unit comprises at least a main electronic circuit 26, supported by an electronic support circuit and by an electronic auxiliary circuit 25.

The main electronic circuit 26 is connected to pushbutton controls 37; 38, 39 which permit to select from the outside the type of instruction to send to the main electronic circuit 26, i.e. the on or off-switching of the device 1, the type of oscillation/rotation of the internal tubular element 4 and the pivotal speed.

Advantageously, a rubber protection 30 can be provided for the aforesaid pushbutton controls 27; 28, 29, placed in a position corresponding to pushbutton controls 37, 38, 39.

The device can have some devices for the control of the speed by the user. To this aim, the device in figure shows five LEDs 36 connected to the control unit in order to indicate the set pivotal speed.

As can be seen in figure 1, the external tubular element through a locknut 35 is connected to the handpiece 2.

Inside the body a group of transmission of motion is also present, comprising a satellite reducer.

In detail, the internal tubular element 4 is brought by a shaft 17 which through a motor pinion 18 functionally connects the internal tubular element with the electric motor 19.

Between the motor pinion 18 and the shaft 17 the box 45 of the satellite reducer is also provided, comprising the satellites 42 and the satellite support shaft 43.

The group for the transmission of motion also has two radial bearings 44, radially juxtaposed, between the motor shaft 17 and the box 45 of the satellite reducer.

The motor pinion 18 engages with the satellites 42 which, through the satellite support shaft 43, transfer the motion to the shaft 17.

Alternatively to the group of coaxial transmission just described a group of transmission is provided with a chain of gears with the provision in any case of the shaft 17 functionally connected to the internal tubular element and through a motor pinion 18 to the electric motor 19.

In this case therefore between the motor pinion 18 and the shaft 17 pinions of first reduction could be provided, a rotary pin of the pinions of first reduction and trimming washers.

The shaft 17 could be pivotally supported by a bearing and a bush, at the distal axial end of the shaft 17.

The electric feeding means 20 of the preferred embodiment shown in figures 1-3 are represented by rechargeable alkaline or lithium batteries, but any other kind of batteries could be used to this aim without departing from the protection field of the invention.

The batteries are contained inside a container 23 provided at the more proximal end of the body.

The container 23 has suitable electric connections in order to feed the electric motor 19 and a non mobile cover for substituting the batteries and for inspection of the electric connections.

Preferably the container 23 is also tight.

The electric motor 19 is housed inside the body 40 which axially extends inside the handpiece 2.

The body 40 centrally contains the motor pinion 18, in a proximal position with respect to the control unit controlling and regulating the motor 19 and frontally the group of transmission of motion.

Preferably, the device 1 according to the present invention can comprise a suction and cooling circuit comprising a connection 9 for a suction apparatus, outside the handpiece 2 and not shown in the figures, at least a duct which guides from said connection 9 the cooling fluid of the internal tubular element 4 and a device for regulating the feeding of the cooling fluid to the internal tubular element.

The device for regulating the feeding of the cooling fluid to the internal tubular element comprises a tap 14 and a lever 13 to control the tap 14 from outside. Advantageously the cooling circuit has a heat exchange portion with said electric motor 19 in order to limit its heating.

To this aim, the heat exchange portion axially extends inside the handpiece 1 in order to axially pass through the entire motor 19.

According to the present invention the handpiece 2 is tight.

According to an alternative aspect of the present invention the feeding means are placed inside the handpiece, and in this case it is possible for example to provide a connection for an external electric source, such as a socket.

According to another aspect of the present invention the entire device 1 is disposable and single-used, in order to guarantee the perfect sterilization of the instrument.

Furthermore, it can be avoided that the personnel of the operation room which performs the treatment and management of the device must perform the washing (with suitable disinfectants and detergents) and then the sterilization of parts of the device.

The personnel of the operation room must not perform the storing of the device in suitable containers which can guarantee the sterilization, with a consequent consumption of time and space.

Nevertheless the personnel of the operation room must not perform anymore the maintenance of non sterilizable components.

## Claims

1. A device (1) for treatments of endoscopic resection/removal of tissues, comprising:
- a handpiece apt to be held by an user;
- an external tubular element (3) comprising a proximal end, a distal end and a cutting aperture disposed at said distal end;
- an internal tubular element (4) apt to be pivotally received in said external tubular element (3) and comprising a proximal end, a distal end and a cutting tip at its distal end;
- wherein the proximal end of each of the external tubular element (3) and internal tubular element (4) are supported by the handpiece
- guide means (5) for rotating and/or oscillating said inner tubular element (4) with respect to said external tubular element (3); wherein said guide means (5) comprise an electric motor (19), a control unit and electric feeding means (20) for said electric motor (19), said guide means (5) being contained inside the handpiece (2); wherein said control unit comprises at least a main electronic circuit (26), to regulate the functions and the speed of the electric motor and of a plurality of pushbutton controls placed on the external surface of said guide means, in a position corresponding to pushbuttons provided on the external surface of the handpiece and
**characterized in that**
said guide means (5) are contained inside a body (40) insertable in a removable way inside said handpiece, said body (40) being tight; the body (40) axially extends inside the handpiece and
only said handpiece (2) is disposable.

2. The device (1) according to claim 1, **characterized in that** said handpiece comprises a distal portion supporting said external and internal tubular elements and a proximal portion engageable in a non mobile way with said distal portion.

3. The device (1) according to any of the preceding claims, **characterized in that** it comprises a group for the transmission of motion from said electric motor (19) in order to rotate said internal tubular element (4) with respect to said external tubular element (3).

4. The device (1) according to any of the preceding claims, **characterized in that** said group for the transmission of motion comprises at least a shaft (17) pivotally supporting said internal tubular element (4) and at least a motor pinion (18) to rotate, actuated by said motor (19), said shaft (17).

5. The device (1) according to any of the preceding claims, **characterized in that** it comprises a cooling circuit comprising a connection for a suction apparatus, at least a duct which guides the cooling fluid to said internal tubular element (4) and a device for regulating the motion.

6. The device according to claim 5, **characterized in that** said cooling circuit has a portion of heat exchange (41) with said electric motor (19) to limit the heating of said electric motor (19).

7. The device according to any of the preceding claims from 5 to 6, **characterized in that** said device for regulating the suction comprises a tap (14) and a lever (13) to control from outside said tap (14).

8. The device according to any of the preceding claims, **characterized in that** said electric motor (19) is a brushless motor.

## Patentansprüche

1. Vorrichtung (1) für Behandlungen von endoskopischer Resektion / Entfernung von Geweben, umfassend:
- ein Handstück, das von einem Benutzer gehalten werden kann;
- ein äußeres röhrenförmiges Element (3), umfassend ein proximales Ende, ein distales Ende und eine Schneidöffnung, die an dem distalen Ende angeordnet ist;
- ein inneres röhrenförmiges Element (4), das in dem äußeren röhrenförmigen Element (3) schwenkbar aufgenommen werden kann und ein proximales Ende, ein distales Ende und eine Schneidspitze an seinem distalen Ende aufweist;
- wobei das proximale Ende von jedem der äußeren röhrenförmigen Elemente (3) and inneren röhrenförmigen Elemente (4) durch ein Handstück getragen werden;
- Führungsmittel (5) zum Drehen und / oder Oszillieren des inneren röhrenförmigen Elements (4) in Bezug auf das äußere röhrenförmigen Element (3);
wobei die Führungsmittel (5) einen Elektromotor (19), eine Steuereinheit und eine elektrische Zuführeinrichtung (20) für den Elektromotor (19) umfasst, wobei die Führungsmittel (5) innerhalb des Handstücks (2) enthalten sind; wobei die Steuereinheit mindestens eine elektronische Hauptschaltung (26) umfasst, um die Funktionen und die Geschwindigkeit des Elektromotors und von einer Vielzahl von an der Außenfläche der Führungsmittel platzierten Drucktastensteuerungen zu steuern, in einer Position die an der Außenfläche des Handstückes bereitgestellten Drucktasten entspricht, und
**dadurch gekennzeichnet, dass**
die Führungsmittel (5) innerhalb eines Körpers (40) enthalten sind, einsetzbar auf eine entfernbare Weise innerhalb des Handstücks, wobei der Körper (40) dicht ist; der Körper (40) ragt axial in das Handstück hinein und
nur das Handstück (2) ist wegwerfbar.

2. Vorrichtung (1) nach Anspruch 1, **dadurch gekennzeichnet, dass** das Handstück einen distalen Abschnitt umfasst, der die äußeren und inneren röhrenförmigen Elemente trägt, und einen proximalen Abschnitt umfasst, der auf eine nicht bewegliche Weise mit dem distalen Abschnitt in Eingriff stehen kann.

3. Vorrichtung (1) nach einem der vorhergehenden Ansprüche, **dadurch gekennzeichnet, dass** sie eine Gruppe zur Bewegungsübertragung von dem Elektromotor (19) umfasst, um das innere röhrenförmige Element (4) in Bezug auf das äußere röhrenförmige Element (3) zu drehen.

4. Vorrichtung (1) nach einem der vorhergehenden Ansprüche, **dadurch gekennzeichnet, dass** die Gruppe zur Bewegungsübertragung mindestens eine Welle (17) umfasst, die das innere röhrenförmige Element (4) schwenkbar trägt, und mindestens ein Motorritzel (18) umfasst, um die Welle (17), betätigt durch den Motor (19), zu drehen.

5. Vorrichtung (1) nach einem der vorhergehenden Ansprüche, **dadurch gekennzeichnet, dass** sie einen Kühlkreislauf umfasst, der eine Verbindung für eine Saugvorrichtung, mindestens einen Kanal, der das Kühlfluid zu dem inneren röhrenförmigen Element (4) führt, und eine Vorrichtung zum Steuern der Bewegung umfasst.

6. Vorrichtung nach Anspruch 5, **dadurch gekennzeichnet, dass** der Kühlkreislauf einen Abschnitt des Wärmeaustauschs (41) mit dem Elektromotor (19) aufweist, um die Erwärmung des Elektromotors (19) zu begrenzen.

7. Vorrichtung nach einem der vorhergehenden Ansprüche von 5 bis 6, **dadurch gekennzeichnet, dass** die Vorrichtung zum Steuern des Saugens einen Hahn (14) und einen Hebel (13) umfasst, um den Hahn (14) von außerhalb zu steuern.

8. Vorrichtung nach einem der vorhergehenden Ansprüche, **dadurch gekennzeichnet, dass** der Elektromotor (19) ein bürstenloser Motor ist.

## Revendications

1. Dispositif (1) pour des traitements de résection/extraction endoscopique de tissus, comprenant :
- une pièce à main qui convient pour être tenue par un utilisateur ;
- un élément tubulaire externe (3) comprenant une extrémité proximale, une extrémité distale et un orifice de découpe disposé au niveau de ladite extrémité distale ;
- un élément tubulaire interne (4) qui convient pour être reçu de manière pivotante dans ledit élément tubulaire externe (3) et comprenant une extrémité proximale, une extrémité distale et un embout de découpe au niveau de son extrémité distale ;
- dans lequel l'extrémité proximale de chacun de l'élément tubulaire externe (3) et de l'élément tubulaire interne (4) est supportée par la pièce à main,
- un moyen de guidage (5) pour faire tourner et/ou faire osciller ledit élément tubulaire interne (4) par rapport audit élément tubulaire externe (3) ;
dans lequel ledit moyen de guidage (5) comprend un moteur électrique (19), une unité de commande et un moyen d'alimentation électrique (20) pour ledit moteur électrique (19), ledit moyen de guidage (5) étant contenu à l'intérieur de la pièce à main (2) ;
dans lequel ladite unité de commande comprend au moins un circuit électronique principal (26), pour réguler les fonctions et la vitesse du moteur électrique et d'une pluralité de commandes de boutons-poussoirs placées sur la surface externe dudit moyen de guidage, dans une position correspondant à des boutons-poussoirs disposés sur la surface externe de la pièce à main et
**caractérisé en ce que**
ledit moyen de guidage (5) est contenu à l'intérieur d'un corps (40) pouvant être inséré de manière amovible à l'intérieur de ladite pièce à main, ledit corps (40) étant étroit ; le corps (40) s'étend de manière axiale à l'intérieur de la pièce à main et
seule la pièce à main (2) est à usage unique.

2. Dispositif (1) selon la revendication 1, **caractérisé en ce que** ladite pièce à main comprend une partie distale supportant lesdits éléments tubulaires externe et interne et une partie proximale pouvant venir en prise de manière non mobile avec ladite partie distale.

3. Dispositif (1) selon l'une quelconque des revendications précédentes, **caractérisé en ce qu'**il comprend un groupe pour la transmission d'un mouvement à partir dudit moteur électrique (19) afin de faire tourner ledit élément tubulaire interne (4) par rapport audit élément tubulaire externe (3).

4. Dispositif (1) selon l'une quelconque des revendications précédentes, **caractérisé en ce que** ledit groupe pour la transmission d'un mouvement comprend au moins un arbre (17) supportant de manière pivotante ledit élément tubulaire interne (4) et au moins un pignon moteur (18) pour faire tourner, actionné par ledit moteur (19), ledit arbre (17).

5. Dispositif (1) selon l'une quelconque des revendications précédentes, **caractérisé en ce qu'**il comprend un circuit de refroidissement comprenant une connexion pour un appareil d'aspiration, au moins une conduite qui guide le fluide de refroidissement vers ledit élément tubulaire interne (4) et un dispositif pour réguler le mouvement.

6. Dispositif selon la revendication 5, **caractérisé en ce que** ledit circuit de refroidissement présente une partie d'échange de chaleur (41) avec ledit moteur électrique (19) pour limiter l'échauffement dudit moteur électrique (19).

7. Dispositif selon l'une quelconque des revendications précédentes de 5 à 6, **caractérisé en ce que** ledit dispositif pour réguler l'aspiration comprend un robinet (14) et un levier (13) pour commander, depuis l'extérieur, ledit robinet (14).

8. Dispositif selon l'une quelconque des revendications précédentes, **caractérisé en ce que** ledit moteur électrique (19) est un moteur sans balais.
